(19) Europäisches Patentamt
European Patent Office
Office européen des brevets

(11) Numéro de publication : **0 382 619 B1**

# (12) FASCICULE DE BREVET EUROPEEN

(45) Date de publication du fascicule du brevet : **06.05.92 Bulletin 92/19**

(51) Int. Cl.$^5$ : **A61K 7/00,** A61K 9/127

(21) Numéro de dépôt : **90400307.6**

(22) Date de dépôt : **05.02.90**

## (54) Procédé de fabrication de mousses utilisables dans les domaines cosmétique et pharmaceutique et mousses obtenues par ceprocédé.

(30) Priorité : **09.02.89 LU 87449**

(43) Date de publication de la demande : **16.08.90 Bulletin 90/33**

(45) Mention de la délivrance du brevet : **06.05.92 Bulletin 92/19**

(84) Etats contractants désignés : **AT BE CH DE DK ES FR GB GR IT LI NL SE**

(56) Documents cités :
**EP-A- 0 190 926**
**WO-A-86/01714**
**FR-A- 2 597 367**
**GB-A- 2 189 457**

(73) Titulaire : **L'OREAL**
**14, Rue Royale**
**F-75008 Paris (FR)**

(72) Inventeur : **Griat, Jacqueline**
**11, Quai de la Baronnie**
**F-94480 Ablon (FR)**
Inventeur : **Ayache, Liliane**
**277, rue de Charenton**
**F-75012 Paris (FR)**

(74) Mandataire : **Peuscet, Jacques et al**
**Cabinet Peuscet 68, rue d'Hauteville**
**F-75010 Paris (FR)**

EP 0 382 619 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé de fabrication de mousses utilisables dans les domaines cosmétique et pharmaceutique et les mousses obtenues par ce procédé.

Dans de nombreux cas, il est avantageux dans le domaine cosmétique ou pharmaceutique d'utiliser des produits sous forme de mousse. Ils sont généralement plus faciles à appliquer car ils sont moins denses et s'étalent plus facilement.

De façon générale, les mousses sont générées à partir d'une phase liquide par action d'un gaz. On introduit, dans une enceinte, qui résiste à la pression et qui est munie d'une valve et d'un ajutage de sortie, le produit liquide à faire mousser et un propulseur pressurisé. On ouvre ensuite la valve de façon à faire expulser la phase liquide sous l'action de la pression générée par le propulseur ; dans le récipient, le propulseur est liquide et il est mélangé au produit liquide à distribuer pour constituer la phase liquide expulsée ; à l'arrivée dans l'atmosphère, le propulseur se détend et forme une mousse dès lors que le produit à distribuer a des caractéristiques moussantes.

Par ailleurs, on sait que certains lipides possèdent la propriété de former, en présence d'eau, des phases mésomorphes, dont l'état d'organisation est intermédiaire entre l'état cristallin et l'état liquide. Parmi les lipides,qui donnent naissance à des phases mésomorphes, il a déjà été indiqué que certains peuvent gonfler en solution aqueuse et former, par agitation, des sphérules (ou vésicules) dispersées dans le milieu aqueux, ces sphérules étant délimitées par des couches multimoléculaires ou bimoléculaires.

Dans les brevets français n° 2 315 991 et 2 543 018, on a déjà décrit des dispersions dans une phase aqueuse D de sphérules ou vésicules lipidiques ; ces vésicules sont caractérisées par leur structure en feuillets constituée d'une ou plusieurs couches lipidiques séparées les unes des autres par des couches de phase aqueuse E ; elles peuvent ainsi servir à encapsuler des substances actives hydrosolubles, par exemple pharmaceutiques ou cosmétiques, et à les protéger des conditions extérieures. Si les composés lipidiques utilisés pour constituer de telles sphérules sont des composés ioniques, il s'agit de liposomes, et s'ils sont des composés non-ioniques, il s'agit de niosomes.

Dans les brevets français 2 485 921 et 2 490 504, on a décrit le fait que la présence de niosomes permet de stabiliser des dispersions en phase aqueuse de liquides non-miscibles à l'eau, en particulier d'huile, sans qu'il soit nécessaire d'ajouter un agent émulsifiant.

Dans les documents WO 86/01714 et EP-A-0 190 926 sont décrits des procédés de préparation de liposomes par pulvérisation de la phase lipidique sous l'action d'un agent propulseur. Dans WO 86/01714 la phase lipidique pulvérisée est amenée en contact avec la surface d'une phase aqueuse pour provoquer la formation des liposomes. Dans EP-A-0 190 926 on pulvérise la phase lipidique en solution dans un solvant organique combiné avec de l'eau, par exemple sous forme d'émulsion.

La présente invention a pour but un procédé de préparation de mousses, utilisables dans les domaines cosmétique et pharmaceutique, à partir de dispersions dans l'eau de liquides non-miscibles à l'eau, en particulier d'huiles, stabilisées par des niosomes.

La présente invention concerne un procédé de préparation de mousses utilisables dans les domaines cosmétique ou pharmaceutique, dans lequel on charge, dans une enceinte qui résiste à la pression et qui est munie d'une valve, un produit cosmétique ou pharmaceutique fluide susceptible de former une mousse et un propulseur pressurisé, puis on ouvre la valve pour éjecter par détente du propulseur le produit cosmétique ou pharmaceutique sous forme de mousse, caractérisé par le fait que l'on utilise, en tant que composant du produit pharmaceutique ou cosmétique, une dispersion d'une phase non-miscible à l'eau dans une phase aqueuse (D), stabilisée par des niosomes constitués d'une ou plusieurs couches de composés lipidiques non-ioniques encapsulant une phase aqueuse (E), cette dispersion constituant 98 à 40% en poids de la charge de l'enceinte, le reste de la charge étant constitué de 2 à 60% en poids de propulseur.

La présente invention concerne également la mousse cosmétique ou pharmaceutique obtenue par ce procédé.

La demanderesse a découvert que, bien que les niosomes soient des structures facilement déstabilisées par des solvants de bas poids moléculaire et que les dispersions stabilisées par ces niosomes soient donc fragiles en présence de ces solvants, de façon surprenante ni les niosomes, ni la dispersion ne sont détruits par action du propulseur.

Selon l'invention, le propulseur peut être un hydrocarbure saturé ayant 3 ou 4 atomes de carbone, tel que le butane, l'isobutane, le propane, ou un hydrocarbure halogéné tels que ceux commercialisés sous la dénomination commerciale "Fréons", ou un mélange de ces composés.

Les lipides utilisés pour la fabrication des niosomes sont des amphiphiles non-ioniques d'origine naturelle ou synthétique comportant, par molécule,une ou plusieurs longue(s) chaîne(s) hydrocarbonée(s). Avantageusement, les composés lipidiques non-ioniques utilisés pour la fabrication des niosomes sont choisis dans le

groupe formé par :

– les éthers de polyglycérol linéaires ou ramifiés de formules :

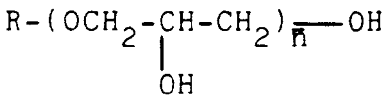 et 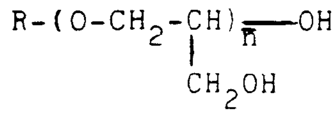

où $\bar{n}$ est une valeur statistique moyenne comprise entre 2 et 6 et R est une chaîne aliphatique linéaire ou ramifiée contenant 16 à 20 atomes de carbone ou le radical hydrocarboné des alcools de lanoline ;

– les éthers de polyglycérol linéaires ou ramifiés contenant deux chaînes grasses ;

– les esters de polyglycérol et d'acides gras à chaîne linéaire ;

– les stérols polyoxyéthylénés ;

– les glycolipides d'origine naturelle ou synthétique, par exemple les cérébrosides et les $\alpha$ ou $\beta$ glucosides d'alcools gras.

De façon connue, divers autres additifs peuvent être associés aux composés lipidiques en vue de modifier la perméabilité ou la charge superficielle des niosomes. On citera, à cet égard, l'addition éventuelle des alcools et diols à longue chaîne, des stérols, par exemple le cholestérol et le $\beta$-sitostérol, des amines à longue chaîne et leurs dérivés ammonium quaternaires, en particulier le bromure de didodécyldiméthylammonium, des bis-hydroxyalkylamines, des amines grasses polyoxyéthylénées ou leurs sels, en particulier leurs dérivés ammonium quaternaires, des esters d'amino-alcools à longue chaîne ainsi que leurs sels et dérivés ammonium quaternaires, des esters phosphoriques d'alcools gras, par exemple le dicétyl- ou le dimyristyl- phosphate de sodium, des alcoylsulfates, par exemple le cétylsulfate de sodium, et des dérivés ioniques des stérols, tels que les phosphates et sulfates de cholestérol.

On peut également ajouter aux composés lipidiques formant des sphérules au moins un lipoprotide, choisi parmi les dérivés mono ou polyacylés d'aminoacides ou de polypeptides dans lesquels le reste acyle R-CO comporte une chaîne hydrocarbonée en $C_{13}$-$C_{19}$, au moins une des fonctions qui relie la chaîne polypeptidique ou le reste d'aminoacide à la chaîne lipophile étant une fonction amide. Le (ou les) lipoprotide(s) est (ou sont) présent(s) à raison de 1 à 15% en poids par rapport au poids total de composés lipidiques proprement dits. Les lipoprotides avantageusement utilisés sont le lipoaminoacide palmitoyl-collagénique, l'acide dipalmitoyl-O-N-hydroxyprolinique et le linoléate d'hydroxyproline.

Avantageusement, on peut utiliser, pour constituer la dispersion de niosomes, de 0,5 à 25% en poids d'amphiphile(s) non-ionique(s) par rapport au poids total de la dispersion de niosomes à obtenir.

Les niosomes utilisés sont, de préférence, des sphérules ayant un diamètre moyen compris entre 100 et 50.000 Å telles que celles décrites dans le brevet français 2 315 991.

On peut prévoir que la phase aqueuse E encapsulée dans les niosomes soit une solution aqueuse de substance cosmétique ou pharmaceutique active, de préférence isoosmotique par rapport à la phase D de la dispersion.

Pour une composition cosmétique, la phase aqueuse E encapsulée dans les niosomes contient par exemple, sous forme dissoute ou sous forme de suspension, au moins un produit pris dans le groupe formé par les humectants, tels que la glycérine, le sorbitol, le pentaérythritol, l'inositol, l'acide pyrrolidone-carboxylique et ses sels, les hydrolysats d'élastine ; les agents de brunissage artificiel, tels que la dihydroxy-acétone, l'érythrulose, le glycéraldéhyde, les alpha-dialdéhydes, tels que l'aldéhyde tartrique, éventuellement associés à des colorants ; les agents anti-solaires hydrosolubles ; les anti-perspirants ; les déodorants ; les astringents ; les produits rafraîchissants, toniques, cicatrisants, kératolytiques, dépilatoires ; les extraits de tissus animaux ou végétaux ; les eaux parfumées ; les colorants hydrosolubles ; les agents anti-pelliculaires ; les agents anti-séborrhéiques ; les oxydants tels que l'eau oxygénée et les réducteurs tels que l'acide thioglycolique et ses sels.

Dans le cas d'une composition utilisable en pharmacie, la phase aqueuse E encapsulée dans les niosomes contient, de préférence, au moins un produit pris dans le groupe formé par les vitamines, les hormones, les enzymes tels que la superoxyde dismutase, les vaccins, les anti-inflammatoires tels que l'hydrocortisone, les antibiotiques, les bactéricides, les antifongiques, les agents antichute ou favorisant la repousse des cheveux et les rétinoïdes.

Dans la composition selon l'invention, la phase aqueuse D entourant les niosomes contient sous forme de dispersion au moins une phase liquide non-miscible à l'eau. Cette phase liquide non-miscible à l'eau est, de préférence, une huile.

L'huile utilisée pour être dispersée dans la phase aqueuse D est avantageusement prise dans le groupe

formé par les esters d'acide gras et de polyol, notamment les triglycérides liquides, et les esters d'acide gras et d'alcool ramifié de formule $R_4$-COOR$_5$, dans laquelle $R_4$ représente le reste d'un acide gras supérieur comportant de 8 à 20 atomes de carbone et $R_5$ représente une chaîne hydrocarbonée ramifiée contenant de 3 à 20 atomes de carbone. Si l'huile est un ester d'acide gras et de polyol, on préfère qu'elle soit choisie dans le groupe formé par les huiles de tournesol, de maïs, de soja, de courge, de pépins de raisin, de sésame, de macadamia, de bourrache, de cassis, et le tricaprocaprylate de glycérol ; si l'huile est un ester d'acide gras supérieur et d'alcool ramifié, on préfère que ce soit l'huile de Purcellin ; d'autres huiles végétales peuvent être utilisées comme, par exemple, l'huile de jojoba.

Le liquide non-miscible à l'eau peut également être :

- un hydrocarbure, tel que l'hexadécane, l'huile de paraffine ou le perhydrosqualène ;
- un hydrocarbure halogéné, tel que le perfluorodécahydronaphtalène ;
- un polysiloxane ;
- un ester d'acide organique ;
- un éther ou un polyéther ;
- ou la perfluorotributylamine.

On peut également prévoir que la phase aqueuse D, qui entoure les niosomes, contienne au moins un adjuvant pris dans le groupe formé par les opacifiants, les gélifiants, les arômes, les filtres solaires hydrosolubles et les colorants. Les adjuvants, qui sont liposolubles, peuvent être dissous dans l'huile non-miscible à l'eau dispersée dans la phase aqueuse D. Si ce liquide non-miscible à l'eau doit contenir des adjuvants dissous, la dissolution de ces adjuvants est, de préférence, réalisée avant d'effectuer la dispersion.

De tels adjuvants peuvent être, par exemple, des filtres solaires liposolubles, tels que le paradiméthylaminobenzoate de 2-éthyl-hexyle, ces parfums ou des substances destinées à améliorer l'état des peaux sèches ou séniles, en particulier des insaponifiables tels que des insaponifiables de soja, d'avocat, des tocophérols, des vitamines E, F, des anti-oxydants.

Les additifs non-liposolubles sont généralement ajoutés à la préparation après la dispersion de l'huile. Le gélifiant peut être introduit à une concentration variant entre 0,1 et 2%, ces pourcentages étant exprimés en poids par rapport au poids total de la composition. Parmi les gélifiants utilisables, on peut citer les dérivés de cellulose, tels que l'hydroxyéthylcellulose ; des dérivés d'algues, tels que celui vendu sous le nom commercial "Satiagum" ; ou encore des gommes naturelles, telles que l'adragante. On préfère utiliser, à titre de gélifiant, l'hydroxyéthylcellulose, un mélange d'acides carboxyvinyliques disponibles dans le commerce sous le nom de "CARBOPOL 940", le produit vendu sous le nom commercial "Satiagum" ou encore de l'adragante.

La phase aqueuse D de dispersion des niosomes et/ou la phase aqueuse interne E des niosomes peut également contenir en solution un polymère polyamide hydrosoluble ayant un poids moléculaire compris entre 1.000 et 200.000, dont la concentration est de 0,01 à 5% en poids par rapport au poids total de la composition. Comme polymère polyamide, on peut citer le polyacrylamide, la poly-bêta-alanine, le poly(acide glutamique), la polytyrosine, la polylysine, et le poly(acide aspartique) et des protéines telles que l'alpha-lactalbumine, le sérumalbumine, les hydrolysats lactiques, les hydrolysats de collagène et les hydrolysats de gélatine.

Pour réaliser la dispersion dans la phase aqueuse D des sphérules lipidiques, on peut utiliser n'importe lequel des procédés antérieurement connus et décrits.

On peut, par exemple, utiliser le procédé, qui consiste à dissoudre les lipides dans un solvant volatil, à former un film mince de lipides sur les parois d'un flacon par évaporation du solvant, à introduire dans ledit flacon la phase aqueuse E à encapsuler et à agiter le mélange mécaniquement jusqu'à l'obtention de la dispersion de sphérules à la taille désirée ; dans ce cas, les phases aqueuses D et E sont nécessairement identiques.

On peut aussi utiliser le procédé décrit dans le brevet français n° 2 315 991. Ce procédé convient particulièrement bien, lorsque l'on souhaite utiliser des sphérules multilamellaires.

Dans le cas où l'on désire des sphérules unilamellaires, on peut utiliser, pour leur préparation, le procédé décrit dans le brevet français n° 2 543 018.

Les exemples donnés ci-dessous, permettront de mieux comprendre l'invention.

## PREPARATION D'UNE DISPERSION A

On prépare une première dispersion A constituée par une dispersion d'huile dans une phase aqueuse, ladite dispersion étant stabilisée par des niosomes.

### 1ère phase :

On utilise le procédé décrit dans le brevet français n° 2 315 991 pour obtenir une dispersion de niosomes

dans une phase aqueuse à partir de la formulation suivante :

- Lipide amphiphile non-ionique de formule :

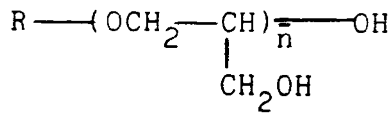

  formule dans laquelle R est un radical hexadécyle et $\bar{n}$ a une valeur statistique moyenne égale à 3...... 3,6 g
- Cholestérol.................................. 3,6 g
- Acide palmitoyl collagénique.............. 0,8 g
- Parahydroxybenzoate de méthyle.................................. 0,3 g
- Glycérine.................................. 3,0 g
- Eau déminéralisée.......................... 35,5 g

On obtient une dispersion de niosomes ayant un diamètre moyen de 10.000 Å.

<u>2ème phase :</u>

On ajoute au mélange ainsi obtenu 15 g d'huile de sésame et 0,4 g de parfum. On soumet le tout à une agitation mécanique jusqu'à obtention d'une dispersion homogène très fine.

On ajoute enfin les substances suivantes :

- Acide polyvinylcarboxylique commercialisé sous le nom de "CARBOPOL 940"................. 0,4 g
- Triéthanolamine.......................... 0,4 g
- Eau déminéralisée................... 37,0 g

<u>PREPARATION D'UNE DISPERSION B</u>

On prépare selon le même procédé une seconde dispersion B à partir de la formulation suivante :

- Lipide amphiphile non-ionique de formule :

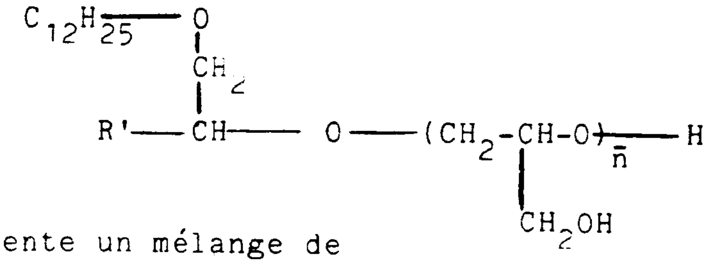

où R' représente un mélange de radicaux $C_{14}H_{24}$ et $C_{16}H_{33}$ et $\bar{n}$ est une valeur statistique moyenne égale à 6....... 0,95 g
- Dimyristyl phosphate........................ 0,05 g
- Parahydroxybenzoate de méthyle........... 0,3 g
- Glycérine.................................. 3 g
- Hydroxyproline............................. 1 g
- Hydrolysats d'élastine.................... 3 g
- Eau déminéralisée.......................... 35,5 g

On ajoute alors à la dispersion de niosomes obtenue les substances suivantes :

- Huile de macadamia......................... 15 g
- Tocophérols................................. 0,2 g
- Parfums...................................... 0,4 g

On soumet le tout à une agitation mécanique jusqu'à obtention d'une dispersion homogène très fine.

On ajoute enfin à la dispersion les substances suivantes :

- Acide polyvinylcarboxylique commercialisé sous le nom de "CARBOPOL 940".... 0,4 g
- Triéthanolamine............................ 0,4 g
- Eau déminéralisée.......................... 39,6 g

EXEMPLES 1 à 5 :

On a introduit les dispersions A ou B préparées comme ci-dessus indiqué comme charge dans un récipient pressurisé avec l'un des quatre gaz propulseurs suivants :

| Dénomination du Propulseur | Composition du mélange formant le propulseur |
|---|---|
| $P_1$ | Butane/Propane/Isobutane (25/20/55) |
| $P_2$ | Difluoro-dichloro-méthane |
| $P_3$ | Difluoro-dichloro-méthane/Dichloro-tétrafluoroéthane (50/50) |
| $P_4$ | $P_1$/Dichlorotétrafluoroéthane (30/70) |

On réalise les cinq exemples suivants correspondant à la mise en oeuvre du procédé selon l'invention :

| N° de l'exemple | Propulseur | | Dispersion | |
|---|---|---|---|---|
| | Nature | Quantité | Nature | Quantité |
| 1 | $P_1$ | 30 | A | 70 |
| 2 | $P_2$ | 30 | A | 70 |
| 3 | $P_1$ | 30 | B | 70 |
| 4 | $P_3$ | 30 | B | 70 |
| 5 | $P_4$ | 30 | B | 70 |
| ces quantités sont données en % en poids par rapport à la charge totale. | | | | |

On constate que, dans tous les cas, les mousses obtenues contiennent des niosomes, qui ont donc subsisté en présence du propulseur et continuent d'avoir dans les mousses leur action de stabilisation des dispersions.

EXEMPLE 6

On a préparé une charge contenant en poids 98 % de la dispersion A et 2 % d'un propulseur constitué d'un mélange propane, isobutane, n-butane (10/50/40).

On a conservé la charge à température ambiante pendant 6 mois et on a vérifié la présence de niosomes par examen ultrastructural en microscopie électronique par la technique de la cryofracture. On a constaté que la charge renferme des niosomes ayant des diamètres compris entre 80 et 300 nm.

EXEMPLE 7

On a stocké pendant 8 mois les charges des exemples 4 et 5 et on a vérifié la présence de niosomes par le procédé décrit dans l' exemple 6. On a constaté la présence de nombreux niosomes ayant un diamètre compris entre 50 et 200 nm dans le cas de la charge de l'exemple 4 et de niosomes ayant un diamètre compris entre 150 et 400 nm dans le cas de la charge de l'exemple 5.

EXEMPLE 8

On a préparé une charge contenant en poids 70 % de dispersion B et 30 % d'un propulseur constitué d'un mélange butane-propane-isobutane-pentane (68,2/8/22/1,8). On a stocké cette charge pendant 8 mois à température ambiance et on a vérifié la présence de niosomes par le procédé décrit à l'exemple 6. On a constaté la présence d'un grand nombre de niosomes ayant un diamètre compris entre 200 et 300 nm.

**Revendications**

1. Procédé de préparation de mousses utilisables dans les domaines cosmétique et pharmaceutique,dans lequel on charge dans une enceinte, qui résiste à la pression et qui est munie d'une valve, un produit cosmétique ou pharmaceutique fluide susceptible de former une mousse et un propulseur pressurisé, puis on ouvre la valve pour faire sortir le produit cosmétique ou pharmaceutique par détente du propulseur de façon à éjecter le produit cosmétique ou pharmaceutique sous forme de mousse, caractérisé par le fait que l'on utilise, en tant que composant du produit cosmétique ou pharmaceutique,une dispersion dans une solution aqueuse (D) d'une phase non-miscible à l'eau stabilisée par des niosomes constitués d'une ou plusieurs couches de composés lipidiques non-ioniques encapsulant une phase aqueuse (E), cette dispersion constituant de 98 à 40% en poids de la charge de l'enceinte, le reste de la charge étant constitué de 2 à 60% en poids de propulseur.

2. Procédé selon la revendication 1, caractérisé par le fait que le propulseur est choisi dans le groupe formé par les hydrocarbures saturés ayant 3 ou 4 atomes de carbone, les hydrocarbures halogénés, et leurs mélanges.

3. Procédé selon l'une des revendications 1 ou 2, caractérisé par le fait que les lipides utilisés pour la fabrication des niosomes sont des amphiphiles non-ioniques d'origine naturelle ou synthétique comportant par molécule une ou plusieurs longue(s) chaîne(s) hydrocarbonée(s).

4. Procédé selon l'une des revendications 1 à 3, caractérisé par le fait que les composés lipidiques non-ioniques sont choisis dans le groupe formé par :

– les éthers de polyglycérol, linéaires ou ramifiés de formules :

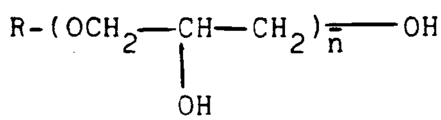

et

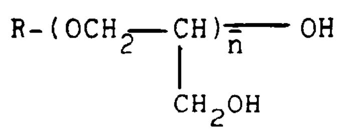

$\bar{n}$ étant une valeur statistique moyenne comprise entre 2 et 6, R étant une chaîne aliphatique linéaire ou ramifiée, comprenant de 16 à 20 atomes de carbone ou le radical hydrocarboné d'un alcool de lanoline ;

– les éthers de polyglycérol linéaires ou ramifiés contenant deux chaînes grasses ;
– les esters de polyglycérol et d'acide gras à chaîne linéaire ;
– les stérols polyoxyéthylénés ;
– les glycolipides d'origine naturelle ou synthétique.

5. Procédé selon l'une des revendications 1 à 4, caractérisé par le fait que l'on utilise de 0,5 à 25 % en poids d'amphiphile(s) non-ionique(s) par rapport au poids total de la dispersion de niosomes à obtenir.

6. Procédé selon l'une des revendications 1 à 5, caractérisé par le fait que l'on ajoute aux composés lipidiques formant les niosomes au moins un composé choisi dans le groupe formé par les alcools et diols à longue chaîne, les stérols, les amines à longue chaîne, les bis-hydroxyalkylamines, les amines grasses polyoxyéthylénées, les esters d'amino-alcools à longue chaîne, leurs sels et leurs dérivés ammonium quaternaires, les esters phosphoriques d'alcools gras, les alcoylsulfates et les dérivés ioniques des stérols.

7. Procédé selon l'une des revendications 1 à 6, caractérisé par le fait que l'on ajoute aux composés lipidiques formant les niosomes au moins un lipoprotide choisi parmi les dérivés mono- ou polyacylés d'aminoacides ou de polypeptides dans lesquels le restes acyle R-CO comporte une chaîne hydrocarbonée en $C_{13}$-$C_{19}$, au moins une des fonctions qui relie la chaîne polypeptidique ou le reste d'amino-acide à la chaîne lipophile étant une fonction amide.

8. Procédé selon la revendication 7, caractérisé par le fait que les lipoprotides sont choisis dans le groupe formé parmi le lipoaminoacide palmitoyl collagénique, l'acide dipalmitoyl-O-N-hydroxyprolinique et le linoléate d'hydroxyproline.

9. Procédé selon l'une des revendications 1 à 8, pour la préparation d'une mousse cosmétique, caractérisé par le fait que la phase aqueuse E encapsulée dans les niosomes contient au moins un produit pris dans le groupe formé par les humectants, tels que la glycérine, le sorbitol, le pentaérythritol, l'inositol, l'acide pyrrolidone carboxylique et ses sels, les hydrolysats d'élastine ; les agents de brunissage artificiel, tels que la dihydroxy-acétone, l'érythrulose, le glycéraldéhyde, les alpha-dialdéhydes, tels que l'aldéhyde tartrique éventuellement associés à des colorants ; les agents anti-perspirants ; les déodorants ; les astringents ; les produits rafraîchissants, toniques, cicatrisants, kératolytiques, dépilatoires ; les extraits de tissus animaux ou végétaux ; les eaux parfumées ; les colorants hydrosolubles ; les agents anti-pelliculaires, les agents anti-solaires ; les agents anti-séborrhéiques ; les oxydants et les réducteurs.

10. Procédé selon l'une des revendications 1 à 8, pour la préparation d'une mousse pharmaceutique, caractérisé par le fait que la phase aqueuse E encapsulée dans les niosomes contient un produit pris dans le groupe formé par les vitamines, les hormones, les enzymes, les vaccins, les anti-inflammatoires, les antibiotiques, les bactéricides, les antifongiques, les agents anti-chute ou favorisant la repousse des cheveux et les rétinoïdes.

11. Procédé selon l'une des revendications 1 à 10, caractérisé par le fait que le liquide non-miscible à l'eau dispersé dans la phase aqueuse D est une huile.

12. Procédé selon la revendication 11, caractérisé par le fait que l'huile est prise dans le groupe formé par les esters d'acide gras et de polyol, et les esters d'acide gras et d'alcool ramifié de formule $R_4$-$COOR_5$, formule dans laquelle $R_4$ représente le reste d'un acide gras supérieur comportant de 8 à 20 atomes de carbone et $R_5$ représente une chaîne hydrocarbonée ramifiée contenant de 3 à 20 atomes de carbone.

13. Procédé selon la revendication 12, caractérisé par le fait que l'huile est un ester d'acide gras et de polyol choisi dans le groupe formé par les huiles de tournesol, de maïs, de soja, de courge, de pépins de raisin, de cassis, de sésame, de macadamia, de bourrache et le tricaprocaprylate de glycérol.

14. Procédé selon la revendication 12, caractérisé par le fait que l'huile est un ester d'acide gras supérieur et d'alcool ramifié constitué par l'huile de Purcellin.

15. Procédé selon l'une des revendications 1 à 10, caractérisé par le fait que le liquide non-miscible à l'eau est un constituant pris dans le groupe formé par les hydrocarbures, les hydrocarbures halogénés, les polysiloxanes, les esters d'acide organique, les éthers et polyéthers et la perfluorotributylamine.

16. Procédé selon l'une des revendications 1 à 15, caractérisé par le fait que la phase D contient un gélifiant.

17. Procédé selon la revendication 16, caractérisé par le fait que le gélifiant est choisi dans le groupe formé par les dérivés de la cellulose, les dérivés d'algue, les gommes naturelles et leurs mélanges.

18. Procédé selon l'une des revendications 1 à 17, caractérisé par le fait que la phase aqueuse D de dispersion des niosomes et/ou la phase aqueuse interne E des niosomes contient en solution au moins un polymère polyamide hydrosoluble ayant un poids moléculaire compris entre 1.000 et 200.000 dont la concentration est comprise entre 0,01 et 5% en poids par rapport au poids total de la dispersion distribuée sous forme de mousse.

19 - Procédé selon la revendication 18, caractérisé par le fait que le polymère polyamide est choisi dans le groupe formé par le polyacrylamide, la poly-bêta-alanine, le poly(acide glutamique), la polytyrosine, la polylysine, et le poly(acide aspartique).

20. Procédé selon la revendication 19, caractérisé par le fait que le polymère polyamide est une protéine choisie dans le groupe formé par l'alpha-lactalbumine, le sérum albumine, les hydrolysats lactiques, les hydrolysats de collagène et les hydrolysats de gélatine.

21. Procédé selon l'une des revendications 1 à 20, caractérisé par le fait que l'on ajoute le liquide non-miscible à l'eau à la phase aqueuse D contenant les niosomes et on soumet le mélange à une agitation intense avant de mélanger avec le propulseur sous pression.

22. Mousse obtenue selon l'une des revendications 1 à 21.

**Patentansprüche**

1. Verfahren zur Herstellung von kosmetisch und pharmazeutisch verwendbaren Schäumen, wobei man

ein druckbeständiges Gefäß, das mit einem Ventil ausgestattet ist, mit einem flüssigen kosmetischen oder pharmazeutischen Produkt, welches zur Schaumbildung befähigt ist, und mit einem unter Druck befindlichen Treibmittel beschickt, anschließend das Ventil öffnet um das kosmetische oder pharmazeutische Produkt durch Ausdehnung des Treibmittels austreten zu lassen, wobei das kosmetische oder pharmazeutische Produkt schaumförmig ausgestoßen wird, **dadurch gekennzeichnet**, daß man als kosmetisches oder pharmazeutisches Produkt eine Dispersion einer mit Wasser nicht mischbaren Phase in einer wässrigen Lösung (D) verwendet, welche durch Niosome stabilisiert ist, die aus einer oder mehreren Schichten nichtionischer Lipidverbindungen bestehen und eine wässrige Phase (E) einschließen, wobei diese Dispersion 98 bis 40 Gew.-% der Füllung des Gefäßes bildet und der restliche Teil der Füllung von 2 bis 60 Gew.-% Treibmittel gebildet wird.

2. Verfahren nach Anspruch 1, **dadurch gekennzeichnet**, daß das Treibmittel ausgewählt ist unter gesättigten Kohlenwasserstoffen mit 3 oder 4 Kohlenstoffatomen, halogenierten Kohlenwasserstoffen und Gemischen davon.

3. Verfahren nach Anspruch 1 oder 2, **dadurch gekennzeichnet**, daß die zur Herstellung der Niosome verwendeten Lipide nichtionische Amphiphile natürlichen oder synthetischen Ursprungs sind und ein oder mehrere langkettige Kohlenwasserstoffmoleküle enthalten.

4. Verfahren nach einem der Ansprüche 1 bis 3, **dadurch gekennzeichnet**, daß die nichtionischen Lipidverbindungen ausgewählt sind unter:

– geradkettigen oder verzweigten Polyglycerinäthern der allgemeinen Formel

$$R-(OCH_2-\underset{\displaystyle OH}{\underset{|}{CH}}-CH_2)_{\bar{n}}-OH$$

und

$$R-(OCH_2-\underset{\displaystyle CH_2OH}{\underset{|}{CH}})_{\bar{n}}-OH$$

worin $\bar{n}$ einen mittleren statistischen Wert von 2 bis 6 einnimmt, R eine geradkettige oder verzweigte aliphatische Kette bedeutet, welche 16 bis 20 Kohlenstoffatome oder den Kohlenwasserstoffrest eines Lanolinalkohols umfaßt;

– geradkettigen oder verzweigten Polyglycerinäthern, welche zwei Fettketten enthalten;
– Estern von Polyglycerin mit geradkettigen Fettsäuren;
– polyoxyethylenierten Sterolen; und
– Glycolipiden natürlichen oder synthetischen Ursprungs.

5. Verfahren nach einem der Ansprüche 1 bis 4, **dadurch gekennzeichnet**, daß man 0,5 bis 25 Gew.-% des (der) nichtionischen Amphiphils (Amphiphile), bezogen auf das Gesamtgewicht der erhaltenen Niosomendispersion, verwendet.

6. Verfahren nach einem der Ansprüche 1 bis 5, **dadurch gekennzeichnet**, daß man zu den die Niosomen bildenden Lipidverbindungen mindestens eine Verindung, ausgewählt unter langkettigen Alkoholen und Diolen, Sterolen, langkettigen Aminen, Bis-hydroxyalkylaminen, polyoxyethylenierten Fettaminen, langkettigen Estern von Aminoalkoholen, den Salzen und quarternären Ammoniumderivaten davon, Phosphorsäureestern von Fettalkoholen, Alkylsulfaten und ionischen Derivaten von Sterolen, hinzugibt.

7. Verfahren nach einem der Ansprüche 1 bis 6, **dadurch gekennzeichnet**, daß man zu den die Niosomen bildenden Lipidveroindungen mindestens ein Lipoproteid, ausgewählt unter Mono- oder Polyacylderivaten von Aminosäuren oder Polypeptiden, worin die Acylreste R-CO eine $C_{13}$ - $C_{19}$-Kohlenwasserstoffkette umfaßen, hinzugibt, wobei mindestens eine der funktionellen Gruppen, welche die Polypeptidkette oder den Aminosäurerest mit der lipophilen Kette verbindet, eine Amidfunktion ist.

8. Verfahren nach Anspruch 7, **dadurch gekennzeichnet,** daß die Lipoproteide ausgewählt sind unter Rollagen- Palmitoyl-Lipoaminosäure , Dipalmitoyl-O-N-hydroxyprolin,(DipalmitoylO-N-hydroxyprolinsäure)und Hydroxyprolinlinoleat.

9. Verfahren nach einem der Ansprüche 1 bis 8, zur Herstellung eines kosmetischen Schaumes, **dadurch**

**gekennzeichnet**, daß die in den Niosomen eingeschlossene wässrige Phase E mindestens ein Produkt enthält, das ausgewählt ist unter Feuchthaltemitteln, wie Glycerin, Sorbitol, Pentaerythritol, Inositol, Pyrrolidoncarbonsäure und dessen Salzen, und Elastinhydrolysaten; künstlichen Bräunungsmitteln, wie Dihydroxyaceton, Erythrulose, Glycerinaldehyd, α-Dialdehyäen, wie Tartronaldehyd, gegebenenfalls in Kombination mit Farbstoffen; Antiperspirantien ; desodorierenden Mitteln; adstringierenden Mitteln; erfrischenden, stärkenden, vernarbenden, keratolytischen, depilatorischen Produkten; Extrakten aus tierischen oder pflanzli'chen Geweben; parfümiertem Wasser; wasserlöslichen Farbstoffen; Antischuppenmitteln; Sonnenschutzmitteln; Antiseborrhoemitteln, C:xidationsmitteln und Reduktionsmitteln.

10. Verfahren nach einem der Ansprüche 1 bis 8, zur Hersteilung eines pharmazeutischen Schaums, **dadurch gekennzeichnet**, daß die in den Niosomen eingeschlossene wässrige Phase E ein Produkt enthält, welches ausgewählt ist unter Vitaminen, Hormonen, Enzymen, Vaccinen, antiinflammatorischen Mitteln, Antibiotika, Bakteriziden, Antifungusmitteln, Mitteln gegen Haarausfall oder zur Förderung des Haarwuchses und Retinoiden.

11. Verfahren nach einem der Ansprüche 1 bis 10, **dadurch gekennzeichnet**, daß das mit Wasser nicht mischbare, in der wässrigen Phase D dispergierte Lipid ein Öl ist.

12. Verfahren nach Anspruch 11, **dadurch gekennzeichnet**, daß das Öl ausgewählt ist unter Estern aus einer Fettsäure und einem Polyol und Estern aus einer Fettsäure und einem verzweigten Alkohol der allgemeinen Formel $R_4$-$COOR_5$, worin $R_4$ den Rest einer höheren Fettsäure mit 8 bis 20 Kohlenstcffatomen bedeutet und $R_5$ eine verzweigte Kohlenwasserstoffkette mit 3 bis 20 Kohlenwasserstoffatomen bedeutet.

13. Verfahren nach Anspruch 12, **dadurch gekennzeichnet**, daß das Öl ein Ester einer Fettsäure mit einem Polyol ist, ausgewählt unter Sonnenblumen-, Mais-, Soja-, Weintraubenkern-, Schwarze Johannisbeeren-, Sesam-, Makadamia-, Gurkenkrautöl und Glycerintricaprocaprylat.

14. Verfahren nach Anspruch 12, **dadurch gekennzeichnet,** daß das Öl eine: Ester einer höheren Fettsäure und eines verzweigten Alkohols Purcellinöl ist.

15. Verfahren nach einem der Ansprüche 1 bis 10, **dadurch gekennzeichnet**, daß das mit Wasser nicht mischbare Lipid eine Verbindung, ausgewählt unter Kohlenwasserstoffen, halogenierten kohlenwasserstoffen, Polysiloxanen, Estern von organischen Säuren, Äthern und Polyäthern und Perfluortributylamin,ist.

16. Verfahren nach einem der Ansprüche 1 bis 15, **dadurch gekennzeichnet**, daß die wässrige Phase D einen Gelbildner enthält.

17. Verfahren nach Anspruch 16, **dadurch gekennzeichnet**, daß der Gelbildner ausgewählt ist unter Celluhsederivaten, Algenderivaten, natürlichen Gummiverbindungen und Gemischen davon.

18. Verfahren nach einem der Ansprüche 1 bis 17, **dadurch gekennzeichnet**, daß die wässrige Phase D der Niosomendispersion und/oder die innere wässrige Phase E der Niosomen in Lösung mindestens ein wasserlösliches Polyamidpolymer enhält, welches ein Molekulargewicht im Bereich von 1000 bis 200000 besitzt, und dessen Konzentration im Bereich von 0,01 bis 5 Gew.-%, bezogen auf das Gesamtgewicht der schaumförmig verteilten Dispersion, liegt.

19. Verfahren nach Anspruch 18, dadurch **gekennzeichnet**, daß das Polyamidpolymer ausgewählt ist unter Polyacrylamid, Poly-β-Alanin, Polyglutaminsäure, Polytyrosin, Polylysin und Polyasparaginsäure.

20. Verfahren nach Anspruch 19, dadurch **gekennzeichnet**, daß das Polyamidpolmer ein Protein ist, ausgewählt unter α-Lactalbumin, Serumalbumin, Milchhydrolysaten, Kollagenhydrolysaten und Gelatinehydrolysaten.

21. Verfahren nach einem der Ansprüche 1 bis 20, dadurch **gekennzeichnet**, daß man das mit Wasser nicht mischbare lipid zu der die Niosomen enthaltenden wässrigen Phase D hinzugibt und das Gemisch kräftig rührt bevor man es mit dem Treibmittel unter Druck vermischt.

22. Schaumförmiges Produkt erhältlich nach einem der Ansprüche 1 bis 21.

## Claims

1. Process for the preparation of foams which can be used in the cosmetics and pharmaceutical fields, in which a chamber which is resistant to pressure and is fitted with a valve is charged with a liquid cosmetic or pharmaceutical product, which can form a foam, and a pressurized propellant and the valve is then opened to discharge the cosmetic or pharmaceutical product by expansion of the propellant such that the cosmetic or pharmaceutical product is ejected in the form of a foam, characterized in that the cosmetic or pharmaceutical product component used is a dispersion of a water-immiacible phase in an aqueous solution (D) stabilized with niosomes consisting of one or more layers of non-ionic lipid compounds encapsulating an aqueous phase (E), this dispersion constituting 98 to 40% by weight of the charge of the chamber, the remainder of the charge consisting of 2 to 60% by weight of propellant.

2. Process according to Claim 1, characterized in that the propellant is chosen from the group comprising saturated hydrocarbons having 3 or 4 carbon atoms, halogenated hydrocarbons and mixtures thereof.

3. Process according to one of Claims 1 or 2, characterized in that the lipids used to prepare the niosomes are nonionic amphiphiles of natural or synthetic origin containing one or more long-chain hydrocarbon(s) per molecule.

4. Process according to one of Claims 1 to 3, characterized in that the nonionic lipid compounds are chosen from the group comprising:

– straight-chain or branched polyglycerol ethers of the formulae:

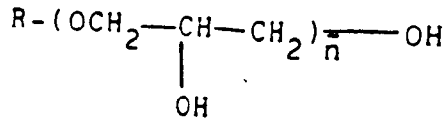

and

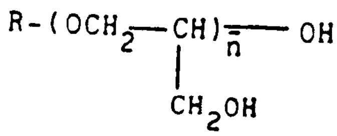

$\bar{n}$ being an average statistical value of between 2 and 6 and R being a linear or branched aliphatic chain containing 16 to 20 carbon atoms or the hydrocarbon radical of a lanolin alcohol;

– straight-chain or branched polyglycerol ethers containing two fatty chains;

– esters of polyglycerol and straight-chain fatty acid;

– polyoxethylenated sterols; and glycolipids of natural or synthetic origin.

5. Process according to one of Claims 1 to 4, characterized in that 0.5 to 25% by weight of nonionic amphiphile(s), with respect to the total weight of the dispersion of niosomes to be obtained, is used.

6. Process according to one of Claims 1 to 5, characterized in that at least one compound chosen from the group comprising long-chain alcohols and diols, sterols, long-chain amines, bis-hydroxyalkylamines, polyoxyethylenated fatty amines, esters of long-chain aminoalcohols, their salts and their quaternary ammonium derivatives, phosphoricesters of fatty alcohols, alkyl-sulphates and ionic derivatives of sterols is added to the lipid compounds forming the niosomes.

7. Process according to one of Claims 1 to 6, characterized in that at least one lipoprotein chosen from the mono- or polyacylated derivatives of amino acids or polypeptides in which the acyl radical R-CO contains a $C_{13}$-$C_{19}$ hydrocarbon chain, at least one of the functions bonding the polypeptide chain or amino acid radical to the lipophilic chain being an amide function, is added to the lipid compounds forming the niosomes.

8. Process according to Claim 7, characterized in that the lipoproteins are chosen from the group comprising collagenic palmitoyl lipoamino acid, O,N-dipalmitoylhydroxyproline and hydroxyproline linoleate.

9. Process according to one of Claims 1 to 8 for the preparation of a cosmetic foam, characterized in that the aqueous phase E encapsulated in the niosomes contains at least one product from the group comprising humectants, such as glycerol, sorbitol, pentaerythritol, inositol, pyrrolidonecarboxylic acid and its salts and elastin hydrolysis products; artificial tanning agents, such as dihydroxyacetone, erythrulose, glyceraldehyde and alpha-dialdehydes, such as tartaric aldehyde, if appropriate combined with colouring agents; antiperspirants; deodorants; astringents; refreshing, tonicizing, cicatrizing, keratolytic and depilatory products; extracts of animal or plant tissues; perfumed waters; water-soluble colouring agents; skin removal agents and antisolar agents; antiseborrhoeic agents; oxidizing agents and reducing agents.

10. Process according to one of Claims 1 to 8 for the preparation of a pharmaceutical foam, characterized in that the aqueous phase E encapsulated in the niosomes contains a product from the group comprising vitamins, hormones, enzymes, vaccines, anti-inflammatory agents, antibiotics, bactericides, antifungal agents, agents to prevent hair loss or promote regrowth of hair and retinoids.

11. Process according to one of Claims 1 to 10, characterized in that the water-immiscible liquid dispersed in the aqueous phase D is an oil.

12. Process according to Claim 11, characterized in that the oil is from the group comprising esters of fatty acid and polyol and esters of fatty acid and branched alcohol, of the formula $R_4$-$COOR_5$, in which formula $R_4$ represents the radical of a higher fatty acid containing 8 to 20 carbon atoms and $R_5$ represents a branched

hydrocarbon chain containing 3 to 20 carbon atoms.

13. Process according to Claim 12, characterized in that the oil is an ester of fatty acid and polyol chosen from the group comprising sunflower, maize, soya, gourd, grape pip, blackcurrant, sesame, macademia and borage oils and glycerol tricaprocaprylate.

14. Process according to Claim 12, characterized in that the oil is an ester of higher fatty acid and branched alcohol constituted by Purcellin oil.

15. Process according to one of Claims 1 to 10, characterized in that the water-immiscible liquid is a constituent from the group comprising hydrocarbons, halogenated hydrocarbons, polysiloxanes, organic acid eaters, ethers and polyethers and perfluorotributylamine.

16. Process according to one of Claims 1 to 15, characterized in that the phase D contains a gelling agent.

17. Process according to Claim 16, characterized in that the gelling agent is chosen from the group comprising cellulose derivatives, algal derivatives, naturally occurring gums and mixtures thereof.

18. Process according to one of Claims 1 to 17, characterized in that the aqueous phase D of the dispersion-of niosomes and/or the aqueous internal phase E of the niosomes contains at least one dissolved water-soluble polyamide polymer having a molecular weight of between 1,000 and 200,000, the concentration of which is between 0.01 and 5% by weight with respect to the total weight of the dispersion distributed in the form of a foam.

19. Process according to Claim 18, characterised in that the polyamide polymer is chosen from the group comprising polyacrylamide, poly-beta-alanine, poly(glutamic acid), polytyrosine, polylysine and poly(sapartic acid).

20. Process according to Claim 19, characterized in that the polyamide polymer is a protein chosen from the group comprising alpha-lactaibumin, serum albumin, lactic hydrolysis products, hydrolysis products of collagen and hydrolysis products of gelatin.

21. Process according to one of Claims 1 to 20, characterized in that the water-immiscible liquid is added to the aqueous phase D containing the niosomes and the mixture is stirred intensively before being mixed with the propellant under pressure.

22. Foam obtained according to one of Claims 1 to 21.